# EUROPÄISCHE PATENTANMELDUNG

(11) **EP 2 186 468 A1**
(43) Veröffentlichungstag der Anmeldung: **19.05.2010**
(21) Anmeldenummer: 09175472.1
(22) Anmeldetag: 10.11.2009
(51) Int. Cl.: A61B 5/00, A61B 5/0205, A61B 5/0408, A61B 5/0416, A61B 5/113

(54) **Gurtsystem**

(30) Priorität: 12.11.2008 DE 102008057597
(71) Anmelder: GETEMED MEDIZIN- UND INFORMATIONSTECHNIK AG, 14513 Teltow (DE)
(72) Erfinder: Müller, Jürgen, 39624 Kakerbeck (DE); Dr. von Nettelhorst, Herwig, 14513 Teltow (DE)
(74) Vertreter: Müller, Wolfram Hubertus

(57) **Zusammenfassung**

Die Erfindung betrifft ein Gurtsystem für einen Patienten. Das Gurtsystem umfasst wenigstens einen Gurt (2, 3, 4a, 4b, 5, 13a, 13b 15) und mehrere Steuerungs- und/oder Messleitungen (9, 9a, 9b, 9c, 9d, 9e), die zumindest teilweise in den Gurt integriert und zur Verbindung mit wenigstens einem Sensor (12) vorgesehen und ausgebildet sind sowie mehrere Austrittstellen des Gurts (2, 3, 4, 5, 13, 15), wobei an einer Austrittstelle (11) jeweils eine der Steuerungs- und/oder Messleitungen (9, 9a, 9b, 9d, 9e) aus dem Gurt (2, 3, 4, 5, 13, 15) austritt, wobei ein Abschnitt der Steuerungs- und Messleitungen (9, 9a, 9b, 9c, 9d, 9e) aus der Austrittstelle herausragt. Dabei weist der Gurt wenigstens eine Anordnungsstelle (10) auf, an der ein Sensor lösbar am Gurt befestigbar ist. Wenigstens einer der herausragenden Abschnitte der Steuerungs- und/oder Messleitungen (9, 9a, 9b, 9c, 9d, 9e) ist derart ausgebildet, dass er wahlweise mit einem an einer der Anordnungsstellen (10) des Gurtes (2, 3, 4, 5, 13, 15) angeordneten Sensor (12) oder mit einem nicht an dem Gurt (2, 3, 4, 5, 13, 15), sondern in anderer Weise am Patienten angeordneten Sensor (12) elektrisch verbindbar ist.

## Beschreibung

Die Erfindung betrifft ein Gurtsystem.

Es sind Messvorrichtungen bekannt, die dazu geeignet sind, die physiologischen Parameter von Patienten über längere Zeiträume ohne Unterbrechung zu überwachen. Eine solche medizinische Überwachung physiologischer Parameter ist insbesondere wichtig für von Schlaganfall oder Herzinfarkt bedrohte Patienten sowie für die medizinische Überwachung von Kleinkindern, insbesondere auch von Neugeborenen. Solche Messvorrichtungen können z. B. auch zur Verhinderung des plötzlichen Kindstods eingesetzt werden.

Die WO 93/10706 A1, die DE 693 14 225 T2 und die US 2008/0154110 A1 beschreiben jeweils ein Gurtsystem mit Gurten, die Elektroden zur Messung physiologischer Parameter eines Patienten aufweisen.

Die Druckschriften WO 93/10706 A1 und US 2008/0154110 A1 beschreiben ausschließlich Elektroden, die in oder an Gurten des jeweiligen Gurtsystems angeordnet sind. Die DE 693 14 225 T2 beschreibt ein Gurtsystem bei dem Elektroden am Gurt anordbar sind und zusätzlich Einzelelektroden an Körperstellen des Patienten angebracht sein können.

Der Erfindung liegt die Aufgabe zugrunde, ein Gurtsystem bereitzustellen, dass in flexiblerer Weise als die bekannten Gurtsysteme eine Überwachung physiologischer Parameter eines Patienten ermöglicht.

Diese Aufgabe wird erfindungsgemäß durch ein Gurtsystem mit den Merkmalen des Anspruchs 1 gelöst. Ausgestaltungen der Erfindung sind in den Unteransprüchen angegeben.

Danach umfasst das erfindungsgemäße Gurtsystem wenigstens einen Gurt und mehrere Steuerungs- und/oder Messleitungen, die zumindest teilweise im Gurt integriert sind und jeweils zur Verbindung mit wenigstens einem Sensor vorgesehen und ausgebildet sind. Darüber hinaus umfasst es mehrere Austrittstellen des Gurtes (und/oder der Gurte), wobei ein Abschnitt der Steuerungs- und/oder Messleitungen aus der Austrittstelle herausragt, und der Gurt wenigstens eine Anordnungsstelle aufweist, an der ein Sensor lösbar am Gurt befestigbar ist. Hierbei ist wenigstens einer der herausragenden Abschnitte der Steuerungs- und/oder Messleitungen derart ausgebildet, dass er wahlweise mit einem an dem Gurt befestigten Sensor oder mit einem nicht an dem Gurt, sondern in anderer Weise am Patienten angeordneten Sensor elektrisch verbindbar ist.

Die erfindungsgemäße Lösung stellt damit eine flexible Vorrichtung bereit, die es erlaubt, eine Messung physiologischer Parameter eines Patienten vorzunehmen. Das am Körper des Patienten getragene Gurtsystem führt dazu, dass die Steuerungs- und/oder Messleitungen, die zur Verbindung mit wenigstens einem Sensor vorgesehen und eingerichtet sind, in definierte Lage zum Patienten gebracht sind und übersichtlich und sicher am oder im Gurt geführt werden. Gleichzeitig wird durch die im Gurt vorgesehenen Austrittsstellen für die Steuerungs- und/oder Messleitungen ein variabler und flexibler Einsatz des Gurts ermöglicht, da ein aus einer Austrittstelle jeweils herausragender Abschnitt einer Steuerungs- und/oder Messleitung sowohl dazu verwendet werden kann, in Kontakt und Verbindung zu einem Sensor zu treten, der mit dem Gurtsystem verbunden und/oder in dieses integriert ist, als auch dazu verwendet werden kann, in Kontakt und Verbindung zu einem Sensor zu treten, der nicht Teil des Gurtsystems ist und in anderer Weise am Patienten befestigt ist. Die Integration der Sensoren in das Gurtsystem ist damit ebenso möglich wie die Anordnung der Sensoren in anderer Weise am Patienten, d.h. die teilweise integrierte Kabelführung kann zur Kontaktierung beliebiger Sensoren und Elektroden genutzt werden.

Ein Sensor kann dabei auch unabhängig vom Gurt am Patienten angeordnet sein. Auch Kombinationen sind möglich. Beispielsweise können mindestens ein an einem Gurt angeordneter Sensor und mindestens ein nicht an einem Gurt, sondern in anderer Weise am Patienten angeordneter Sensor vorgesehen sein. Ein erfindungsgemäß aus der Austrittstelle herausragender Abschnitt einer Steuerungs- und/oder Messleitung kann dabei sowohl mit einem Sensor, der an einem Gurt angeordnet ist, als auch mit einem Sensor, der nicht an einem Gurt angeordnet ist, verbunden werden. Dies trägt zu der bereits oben beschriebenen hohen Flexibilität in der Verbindbarkeit des Gurtsystems mit unterschiedlich angeordneten und ausgebildeten Sensoren bei.

Die Verbindung mit einem Sensor über aus dem Gurtsystem austretende Steuerungs- und/oder Messleitungen ist auch deshalb vorteilhaft, weil beispielsweise bei Drehbewegungen des Oberkörpers des Patienten um seine longitudinale Achse die Zugkräfte auf Steuerungs- und/oder Messleitungen gegenüber dem Fall verringert sind, dass kein erfindungsgemäßes Gurtsystem vorhanden wäre, und dann z. B. Messleitungen direkt von einem Auswertgerät zu einem Sensor oder zu Sensoren geführt würden. Damit bewirkt das bestimmungsgemäß angelegte Gurtsystem eine vorteilhafte Zugentlastung der mit Sensoren verbundenen Steuerungs- und Messleitungen.

In einer Ausgestaltung der Erfindung sind alle oder mehrere der Steuerungs- und/oder Messleitungen zu einem Anschluss zusammengeführt, der mit einer externen Steuerungs- und/oder Auswerteinrichtung verbindbar ist. Dieser Anschluss bildet - sofern alle Steuerungs- und/oder Messleitungen zu ihm zusammengeführt werden - eine gemeinsame Schnittstelle dieser Steuerungs- und/oder Messleitungen zu der externen Steuerungs- und/oder Auswerteinrichtung. Dieser Anschluss kann beispielsweise einen Anschlussstecker oder eine Anschlusssteckerbuchse aufweisen. Hierdurch kann eine zentrale Auswertung der vom Sensor oder von Sensoren erfassten Daten extern vorgenommen werden, wenn das Gurtsystem am Patienten angelegt ist und zugeordneten Sensoren, die z. B. physiologische Parameter des Patienten messen, aktiviert sind.

Weiter kann vorgesehen sein, dass alle oder mehrere der Steuerungs- und/oder Messleitungen zumindest in einem zentralen Strang mittels einer Kabelführung am Gurtsystem geführt werden. Dies erhöht die Übersichtlichkeit und Sicherheit der Führung der Leitungen am Gurt. Auch kann vorgesehen sein, dass alle oder mehrere der Steuerungs- und/oder Messleitungen zumindest über einen Teil ihrer Erstreckungslänge verdrillt geführt sind. Dadurch wird die gegenseitige und äußere Beeinflussung der Steuerungs- und/oder Messleitungen durch elektromagnetische und kapazitive Effekte verringert.

Bei einer Ausführungsvariante sind, soweit mehrere dieser Gurte (wie z. B. Schultergurt, Beingurt) vorhanden sind, diese so miteinander verbunden oder verbindbar, dass ein gegen Verrutschen weitgehend gesichertes Anlegen der Gurte an den Körper des Patienten ermöglicht ist.

In einer Ausgestaltung der Erfindung umfasst das Gurtsystem zumindest einen Brustgurt, der um den Oberkörper des Patienten tragbar ist, und/oder einen Bauchgurt, der um den Bauchbereich des Patienten tragbar ist. Brust- und Bauchgurt sind in einer Ausführungsvariante beide vorgesehen. Weiter kann ein Gurtsteg vorgesehen sein, der mit mindestens einem anderen Gurt verbunden ist. Insbesondere kann die Verbindung zwischen Gurtsteg und dem anderen Gurt so erfolgen, dass ein T-förmiger Gurt gebildet wird. Ausführungsvarianten mit mehrere Gurtstegen und Gurten, von denen bestimmte untereinander verbunden oder verbindbar sind, sind ebenfalls vorgesehen.

So kann in einem Ausführungsbeispiel der Gurtsteg einen Brust- und einen Bauchgurt miteinander verbinden. Dies führt zu einer Stabilisierung des Gurtsystems. Des Weiteren können über den Gurtsteg, z. B. in einer Kabelführung, Steuerungs- und/oder Messleitungen zwischen dem Bauch- und Brustgurt geführt werden. Ein Vorteil ist, dass auf diese Weise eine stabile Führung dieser Steuerungs- und/oder Messleitungen zwischen Bauch- und oder Brustgurt erfolgt.

In einer weiteren Ausgestaltung umfasst das Gurtsystem einen Brustgurt, einen Bauchgurt und einen Gurtsteg, der wiederum zwei Abschnitte umfasst, die einen unteren und oberen Gurtsteg bilden. Hierbei ist der obere Gurtsteg mindestens mit dem Bauchgurt und dem Brustgurt verbunden. Der untere Gurtsteg ist mindestens mit dem Bauchgurt verbunden. In einer Ausführungsvariante bilden Bauchgurt, Brustgurt und der obere Gurtsteg einen Gurt in Form eines Doppelt-T. Hierbei ist der untere Gurtsteg, der mit dem Bauchgurt verbunden ist, optional.

Bei einer weiteren Ausgestaltung ist im an den Patienten angelegten Zustand wenigstens ein Teil des Gurtstegs im Wesentlichen in Richtung der longitudinalen Achse des Patienten ausgerichtet. In einer Ausführung ist dabei der untere Gurtsteg dafür eingerichtet und vorgesehen, entlang eines der Beine des Patienten führbar zu sein. Am Ende dieses Abschnitts ist beispielsweise der erwähnte Anschluss zur Verbindung mit einer externen Steuerungs- und/oder Auswerteinrichtung vorgesehen.

In einem Ausführungsbeispiel umfasst das Gurtsystem wenigstens einen Sensor, d.h. ein oder mehrere Sensoren sind am Gurtsystem angeordnet. Beispielsweise ist der Sensor ein Elektrokardiogramm (EKG)-Sensor, wie z. B. eine EKG-Elektrode. Dabei ist ein Sensor jeweils in oder an einem der Gurte befestigt und fixiert. Dies weist den Vorteil auf, dass die Positionierung der Sensoren bei einer Neuanbringung des Gurtes automatisch richtig ist.

Erfindungsgemäß weist der Gurt oder weisen die Gurte des Gurtsystems mehrere Anordnungsstellen auf, die dazu vorgesehen und eingerichtet sind, dass an ihnen ein Sensor anordbar ist. Eine solche Anordnungsstelle wird beispielsweise durch eine dafür vorgesehene Befestigungsvorrichtung am Gurt gebildet. Hierzu können der Gurt und der Sensor lösbare Verschlussmittel zur Befestigung aufweisen. Bei einem Ausführungsbeispiel wird dieses Verschlussmittel durch einen Klettverschluss gebildet. Der Sensor weist hierzu einen Teil des Klettverschlusses auf und ist mit mehreren dafür vorgesehenen und eingerichteten Anordnungsstellen am Gurt verbindbar. Insbesondere kann der Gurt so eingerichtet sein, dass er spezifische Stellen aufweist, mit denen der Teil des am Sensor angeordneten Klettverschlusses verbunden werden kann. Auch die gesamte Unterseite oder große Flächen der Unterseite des Gurtes mit einer Haftfläche eines Klettverschlusses versehen sein. Durch mehrere Anordnungsstellen am Gurt, mit denen der am Sensor vorgesehene Teil des Klettverschlusses lösbar verbindbar ist, kann die Position eines Sensors in einfacher Weise auf diese bestimmten Stellen festgelegt werden. Die Bereiche des Gurtes, an dem der Sensor über einen Klettverschluss verbindbar ist, können gesondert für den Anwender gekennzeichnet sein.

In einer Ausführungsvariante ist eine solche Anordnungsstelle benachbart zu einer Austrittstelle, an der eine der Steuerungs- und/oder Messleitung aus dem Gurt austritt, ausgebildet. Die Steuerungs- und/oder Messleitung kann dabei mit einem am Gurt an einer solchen Anordnungsstelle angeordneten Sensor verbunden werden. Beispielsweise ist eine solche Anordnungsstelle in einem Abstand von 1 bis 10 cm, insbesondere von 2 bis 8 cm zur nächstgelegenen Austrittstelle angeordnet, so dass der außerhalb des Gurtes geführte Abschnitt der Steuerungs- und/oder Messleitung kurz gewählt werden kann.

In einer Ausgestaltung weist mindestens ein Gurt oder Gurtabschnitt des Gurtsystems mehrere Anordnungsstellen zur Anordnung von Sensoren in definierter Lage zueinander auf. Diese Anordnungsstellen spezifizieren die Positionierung des Sensors relativ zum Gurt und Gurtsystem. Hierdurch können definierte Abstände zwischen den Sensoren realisiert werden, wobei die in definierter Lage zueinander angebrachten Anordnungsstellen als Abstandsmaß dienen. Wie bereits beschrieben, trägt eine solche Anordnung auch zur Zugentlastung in dem Fall bei, dass der Patient sich bewegt, da aufgrund der relativ festen Position des Gurtsystems zum Patient und der Anordnung des Sensors am Gurtsystem keine oder geringe Zugkräfte auf die mit Sensoren verbundenen Steuerungs- und/oder Messleitungen wirken.

Insgesamt wird durch die verschiedenen Ausführungsvarianten und ihre Kombinationsmöglichkeiten eine flexible Einsetzbarkeit des Gurtsystems zusammen mit einem oder mehreren Sensoren ermöglicht. So ist es beispielsweise möglich, mehrere Sensoren an den dafür vorgesehenen Stellen an einem oder mehreren der Gurte anzuordnen und darüber hinaus optional noch einen oder mehrere andere Sensoren entfernt von den Gurten anzubringen und alle oder nur einen gewählten Teil der Sensoren mit jeweils einer Steuerungs- und/oder Messleitung des Gurtsystems zu verbinden. Dies bietet Vorteile insbesondere bei der EKG-Messung, da die Messpunkte flexibel festgelegt werden können, die dafür vorgesehenen Stellen hierfür eine feste Orientierungshilfe bieten und das am Patienten angelegte Gurtsystem insgesamt dazu beiträgt, dass die Verbindungen von Steuerungs- und/oder Messleitungen mit dem Sensor oder den Sensoren zugentlastet sind.

In einem weiteren Ausführungsbeispiel umfasst das Gurtsystem einen oder mehrere Widerstandsstreifen, die in oder an mindestens einem Gurt des Gurtsystems angeordnet sind. Es können auch mehrere Widerstandsstreifen vorgesehen sein, die in oder an unterschiedlichen Gurten des Gurtsystems angeordnet sind, so dass mehrere unabhängige Signale erfasst und ausgewertet werden können. In einer Variante sind die Widerstandsstreifen als Teil eines Impedanzmesskreises miteinander verschaltet, beispielsweise eines Impedanzmesskreises zur Messung der Atmungstätigkeit des Patienten. Dies ist vorteilhaft gegenüber einer herkömmlichen Messung der Atmungstätigkeit, bei der die transthorakale Widerstandsänderung des Oberkörpers des Patienten zwischen mehreren Sensoren gemessen wird, was einen wenn auch geringen Stromfluss durch den menschlichen Körper erfordert.

Bei einer Ausführungsvariante weist wenigstens ein Gurt Mittel auf, mittels derer die Länge des Gurtes zur Anpassung an die Körpermaße des Patienten einstellbar ist. Dies ist unter anderem durch Klettverschluss, Schnalle, Zurrvorrichtung oder Ähnliches erreichbar. Vorteilhaft hieran ist, dass das Gurtsystem nacheinander fest am Körper verschiedener Patienten, die unterschiedliche Körpermaße aufweisen, nach jeweils entsprechender Anpassung an die Körpermaße, fixierbar ist.

In einer weiteren Ausführungsform ist wenigstens ein Gurt an mindestens einer dafür vorgesehen Stelle ohne Unterbrechung der Steuerungs- und/oder Messleitungen durch ein Mittel öffenbar und wiederverschließbar. Dies ist unter anderem durch übliche Gurtverschlüsse erreichbar, wie z. B. Klettverschluss oder Verschlussschnalle.

In einem Ausführungsbeispiel weist das Gurtsystem des Weiteren eine Steuerelektronik und/oder eine Stromversorgung für wenigstens einen Sensor auf.

Der Gurt oder die Gurte bilden in einem Ausführungsbeispiel einen einstückigen Gesamtgurt.

Weitere Merkmale und Vorteile der Erfindung werden bei der nachfolgenden Beschreibung von Ausführungsbeispielen anhand der Figuren deutlich werden. Es zeigen:

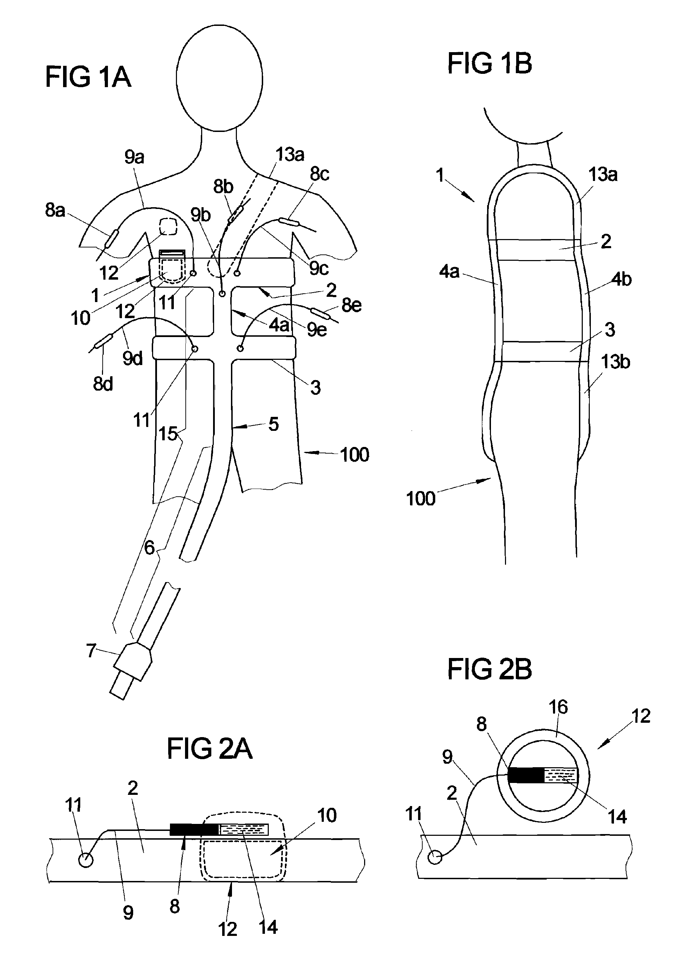

- Fig. 1a: ein Ausführungsbeispiel eines Gurtsystems;
- Fig. 1 b: ein weiteres Ausführungsbeispiel eines Gurtsystems;
- Fig. 2a: einen Ausschnitt eines Gurtsystems mit am Gurtsystem befestigten Sensor; und
- Fig. 2b: einen Ausschnitt eines Gurtsystems, wobei ein Sensor direkt am Patienten angeordnet ist

Die Fig. 1a zeigt in schematischer Darstellung einen Patienten 100, der ein Gurtsystems 1 trägt. Das Gurtsystem 1 umfasst unter anderem einen Brustgurt 2, der auf Brusthöhe des Patienten 100 angelegt ist und der über einen oberen Gurtsteg 4a mit einem Bauchgurt 3 verbunden ist. Die Gurte 2, 3, 4a sind beispielsweise lösbar (etwa durch Druckknöpfe) oder unlösbar (etwa durch Vernähen) miteinander verbunden oder sind als einstückiger Gesamtgurt gefertigt. Der obere Gurtsteg 4a ist im Wesentlichen parallel zur longitudinalen Achse des Patienten 100 angelegt und verbindet den Brustgurt 2 mit dem Bauchgurt 3.

Auf Bauchhöhe des Patienten 100 bilden der obere Gurtsteg 4a, der untere Gurtsteg 5 und der Bauchgurt 3 eine kreuzförmige Verbindung. Auf Brusthöhe bilden der obere Gurtsteg 4a und der Brustgurt 2 eine T-förmige Verbindung. Durch den oberen Gurtsteg 4a und unteren den Gurtsteg 5 sowie den Bauch- und Brustgurt 3, 2 ist eine sichere Fixierung des Gurtsystems 1 am Körper des Patienten 100 gegen Verrutschen möglich.

An einer Thoraxseite des Patienten sind der Brustgurt 2 und der Bauchgurt 3 in sich öffenbar. Hierzu sind im angelegten Zustand Teile, die wenigstens einen Teil des Brust- bzw. Bauchgurt 2, 3 bilden, mit einem Klettverschluss (nicht abgebildet) in sich verbindbar. Hierdurch kann eine Anpassung an die Körpermaße des Patienten 100 unter gleichzeitiger Fixierung des Gurtsystems 1 erreicht werden.

Mit dem Brustgurt 2 ist ein optionaler und daher gestrichelt gekennzeichneter Schultergurt 13a verbindbar, der auf der Rückenseite des Patienten 100 an einer weiteren Stelle mit dem Brustgurt 2 verbunden ist. Dieser optionale Schultergurt führt zu einer weiter verbesserten Fixierung.

Im Brustgurt 2, dem oberen Gurtsteg 4a, dem unteren Gurtsteg 5 und dem Bauchgurt 3 sind Anordnungsstellen 10, von denen eine exemplarisch dargestellt ist, so vorgesehen, dass optionale Sensoren 12 mit Anschlussstecker (nicht abgebildet) an ihnen anordbar sind. Hierzu sind der Sensor 12 und die Anordnungsstellen 10 mit einem Teil eines Klettverschlusses so ausgestattet, dass der Sensor 12 dort mittels des Klettverschlusses mit dem Gurtsystem 1 lösbar verbindbar ist. Dadurch, dass in Ausführungsvarianten mehrere Anordnungsstellen 10 in definierten Abständen in den Gurten ausgebildet sind, wird erreicht, dass die optionalen Sensoren 12 ebenfalls in definierten Abständen zueinander angeordnet werden können.

Es kann dabei vorgesehen sein, dass die gesamte dem Patienten zugewandte Oberfläche zumindest des Brustgurts 2 und des Bauchgurts 3 als Haftfläche eines Klettverschlusses ausgebildet sind.

Auf dem Brustgurt 2, dem Bauchgurt 3 sowie auf dem oberen Gurtsteg 4a und dem unteren 5 Gurtsteg sind des weiteren Austrittstellen 11 für Kabel ausgebildet, die in das Gurtsystem integriert sind. Auf der Seite des Brustgurtes 2, die zur Vorderseite des Patienten 100 weist, sind im dargestellten Ausführungsbeispiel zwei solche Austrittsstellen 11 ausgebildet, die beide Austrittspunkte je eines Anschlusskabels 9a bzw. 9c sind. Der obere Gurtsteg 4a weist eine Austrittsstelle 11 mit einem Anschlusskabel 9b auf. Der untere Gurtsteg weist eine Austrittsstelle 11 auf, aus der kein Anschlusskabel 9 tritt. Der Bauchgurt 3 weist auf der Vorderseite des Patienten 100 zwei solche Austrittsstellen 11 auf, aus denen Anschlusskabel 9d und 9e austreten. Am Ende jedes der Anschlusskabel 9, 9a, 9b, 9c, 9d, 9e ist je eine Anschlusssteckerbuchse 8, 8a, 8b, 8c, 8d, 8e vorgesehen, die beispielsweise über einen korrespondierenden Anschlussstecker eines Sensors 12 mit einem solchen Sensor 12 verbindbar ist.

Jede Austrittsstelle 11, aus der ein Anschlusskabel 9 austritt, kann in der Nähe eine Anordnungsstelle 10 ausgebildet sein, die zur optionalen Anordnung von Sensoren 12 vorgesehen sind. Beim Anordnen von Sensoren 12 an einer dieser Anordnungsstellen 10 und Verbinden dieser Sensoren 12 mit den Anschlusskabeln 9 jeweils über eine Anschlusssteckerbuchse 8 und einen Anschlussstecker 14 (nicht abgebildet) ergibt sich beim Zusammenwirken mit der durch das Gurtsystem gegebenen Fixierung eine stabile Anordnung zum Messen medizinisch relevanter Körperfunktionen. Durch die Fixierung der Sensoren 12 relativ zum Gurtsystem und die Verbindung mit den dafür bestimmten, aus dem Gurtsystem austretenden Steuerungs- und/oder Messleitungen wird dabei auch eine Zugentlastung auf die Verbindung über die Anschlusssteckerbuchse 8 und den Anschlussstecker bereitgestellt. Selbst wenn der Patient z. B. an seinem Oberkörper um seine longitudinale Achse gedreht werden sollte, wirkt hierdurch keine zusätzliche Zuglast auf die Verbindung der Sensoren 12 mit den zugeordneten Steuerungs- und/oder Messleitungen.

Sensoren 12 können alternativ oder zusätzlich - wie beispielhaft abgebildet - auch auf dem Körper des Patienten befestigt sein, ohne dabei direkt an einem der Gurte angeordnet zu sein. Durch Austritt der Anschlusskabel 9 aus dem Gurtsystem an den Austrittsstellen 11 können beliebige und beliebig angeordnete Sensoren 12 kontaktiert werden.

Die Sensoren 12 sind beispielsweise durch dafür vorgesehene Befestigungsmittel, wie z. B. Klebering oder Pflaster als Haftmittel 16, am Patienten angebracht.

Die Zuleitungen zu den Anschlusskabeln 9, 9a, 9c, 9d und 9e sind in dem Gurtsystem integriert geführt. Diese Zuleitungen werden von allen Teilen des Gurtsystems 1 über den unteren Gurtsteg 5 geführt. Zumindest in einem Teilbereich 6 des unteren Gurtstegs 5 sind mehrere oder alle der Zuleitungen verdrillt geführt. Zusätzlich können die Zuleitungen in einem Kabel geführt sein. Eine Verdrillung der Leitungen trägt zu einer gegenseitigen Abschirmung und zu einer Abschirmung gegen äußere Felder bei. Eine Kabelführung erleichtert die Handhabung. Die Abbildung des Teilbereichs 6 in der Fig. 1 ist nur exemplarisch zu verstehen. Eine verdrillte Leitungsführung kann in einer anderen Ausgestaltung insbesondere über einen kleineren oder größeren Bereich der Erstreckungslänge der Steuerungs- und/oder Messleitungen realisiert sein.

Ein Teil des unteren Gurtstegs 5 ist so vorgesehen, dass er unter der Bekleidung (nicht abgebildet) des Patienten 100 entlang eines der Beine des Patienten 100 geführt werden kann. Im unteren Gurtsteg 5 sind alle Verbindungskabel zu und von elektrischen Einrichtungen des Gurtsystems zu einem externen Anschluss mit einem externen Anschlussstecker 7 zusammengeführt. Dieser externe Anschlussstecker 7 ermöglicht den Anschluss des Gurtsystems 1 zur Übertragung von Messwerten auf externe Vorrichtungen wie z. B. eine EKG-Auswertungsvorrichtung und/oder zur Bestromung des Gurtsystems 1 und/oder zur Steuerung der Messeinrichtungen des Gurtsystems 1.

Im abgebildeten Ausführungsbeispiel sind die Mess- und Steuerleitungen 9a, 9b, 9c, 9d mit EKG-Elektroden 12 verbunden. Auf diese Weise kann ein mehrkanaliges Elektrokardiogramm abgeleitet werden. Z. B. kann - einen Anschluss der Mess- und Steuerleitungen 9 an entsprechend angeordnete Elektrokardiogramm-Sensoren vorausgesetzt - ein Kanal 1 des Elektrokardiogramms über die Mess- und Steuerleitungen 9a und 9e und ein Kanal 2 über die Mess- und Steuerleitungen 9c und 9d abgleitet werden, wobei die Mess- und Steuerleitung 9b mit einem Sensor verbindbar ist, der als Bezugspunkt der Elektrokardiogramm-Messung fungieren kann. Die Atmung kann in einem solchen Fall zusätzlich über die Messleitungen 9a, 9e oder 9a bzw. die Messleitungen 9c oder 9d und 9e gemessen werden. Man erhält so die Möglichkeit, die Signale für Atmung und Elektrokardiogramm getrennt zu optimieren.

Zusätzlich können Widerstandsstreifen (nicht abgebildet) in die Gurte integriert werden und die Atmung über diese Widerstandsstreifen und nicht über eine transthorakale Widerstandsänderung des Oberkörpers ermittelt werden. Insbesondere können solche Widerstandsstreifen in den Brustgurt 1 und/oder den Bauchgurt 3 integriert werden. Die Integration von Widerstandsstreifen in beide Gurte 1, 3 weist dabei den Vorteil auf, dass zwei unabhängige Signale zur Atmungsüberwachung vorliegen und ausgewertet werden können.

Die Fig. 1b zeigt in schematischer Darstellung einen Patienten 100, der ein Gurtsystem 1 trägt. Das Gurtsystem umfasst unter anderem einen Brustgurt 2, der auf Brusthöhe des Patienten 100 angelegt ist und der über je einen oberen Gurtsteg 4a, 4b auf der Vorder- und auf der Rückenseite des Patienten 100 mit einem Bauchgut 3 verbunden ist. Außerdem weist die abgebildete Ausführung des Gurtsystems 1 einen Schultergurt 13a und einen Beingurt 13b auf, wobei der Schultergurt über wenigstens eine der Schultern des Patienten so geführt ist, dass er auf der Vorderseite und auf der Rückseite des Patienten 100 jeweils mit dem Brustgurt 2 und dem oberen Gurtsteg verbunden ist. Durch den Schultergurt 13a werden der Brustgurt 2 und die mit dem Brustgurt 2 verbundenen oberen Gurtstege 4a, 4b am Patienten fixiert.

Der Bauchgurt 3 und die oberen Gurtstege 4a, 4b sind in der abgebildeten Ausführung des Gurtsystems 1 mit einem Beingurt 13b verbunden, der zwischen den Beinen des Patienten geführt ist. Hierdurch wird eine Fixierung des Gurtsystems am Patienten erreicht. Die Gurte sind beispielsweise lösbar (z. B. durch Druckknöpfe oder Klettverschlüsse) oder unlösbar (z. B. durch Vernähen) miteinander verbunden oder sind als einstückiger Gesamtgurt gefertigt. Hierbei können auch Kombinationen von Gurtverbindungen auftreten, z. B. können Brustgurt 2 und Bauchgurt 3 mit den oberen Gurtstegen 4a, 4b vernäht sein und der Schultergurt 13a sowie der Beingurt 13b lösbar mit den anderen Gurten 2, 3, 4a, 4b des Gurtsystems 13a, 13b verbindbar sein.

Die Fig. 2a zeigt einen Ausschnitt des Brustgurtes 2 des Gurtsystems 1. Aus dem Brustgurt 2 tritt an der Austrittsstelle 11 ein Anschlusskabel 9 als Zu- und Ableitung für einen Sensor 12, beispielsweise eine EKG-Elektrode, aus. Dieses Anschlusskabel 9 ist über eine Anschlusssteckerbuchse 8 mit einem Anschlussstecker 14 des Sensors 12 verbunden. Der Anschlussstecker 14 ist dabei auf dem Sensor 12 angeordnet und mit diesem verbunden.. Der Sensor 12 ist an einer Anordnungsstelle 10 des Brustgurtes 2 angeordnet. Die Anordnungsstelle 10 ist dabei so eingerichtet, dass ein mit dem Sensor verbundener Teil eines Klettverschlusses dort mit dem Gurt verbunden werden kann. Die Anordnungsstelle 10 liegt dabei in räumlicher Nähe zur Austrittsstelle 11.

Die Fig. 2b zeigt einen Ausschnitt des Brustgurtes 2 des Gurtsystems 1. Aus dem Brustgurt 2 tritt an der Austrittsstelle 11 ein Anschlusskabel 9 als Zu- und Ableitung für einen Sensor 12, beispielsweise eine EKG-Elektrode, aus. Dieses Anschlusskabel 9 ist über eine Anschlusssteckerbuchse 8 mit einem Anschlussstecker 14 des Sensors 14 verbunden. Der Anschlussstecker 14 ist dabei auf der vom Patienten abgewandten Oberseite des Sensors 12 angeordnet. Der Sensor 12 ist auf der Haut des Patienten mittels eines mit dem Sensor verbundenen Klebering als lösbarem Haftmittel 16 angeordnet. Eine Anordnung des Sensors 12 am Gurtsystem 1 ist hier nicht vorgesehen.

## Patentansprüche

1. Gurtsystem für einen Patienten, das
• wenigstens einen Gurt (2, 3, 4a, 4b, 5, 13a, 13b, 15),
• mehrere Steuerungs- und/oder Messleitungen (9, 9a, 9b, 9c, 9d, 9e), die in den Gurt (2, 3, 4, 5, 13, 15) integriert sind und jeweils zur Verbindung mit wenigstens einem Sensor (12) vorgesehen und ausgebildet sind, und mehrere Austrittstellen des Gurts (2,3, 4, 5, 13, 15) umfasst, wobei
• an einer Austrittstelle (11) jeweils eine der Steuerungs- und/oder Messleitungen (9, 9a, 9b, 9d, 9e) aus dem Gurt (2, 3, 4, 5, 13, 15) austritt, wobei ein Abschnitt der Steuerungs- und/oder Messleitung (9, 9a, 9b, 9c, 9d, 9e) aus der Austrittstelle (11) herausragt, der Gurt (2, 3, 4, 5, 13, 15) wenigstens eine Anordnungsstelle (10) aufweist, an der ein Sensor lösbar am Gurt befestigbar ist, und
• wenigstens einer der herausragenden Abschnitte der Steuerungs- und/oder Messleitungen (9, 9a, 9b, 9c, 9d, 9e) derart ausgebildet ist, dass er wahlweise mit einem an einer der Anordnungsstellen (10) des Gurtes (2, 3, 4, 5, 13, 15) angeordneten Sensor (12) oder mit einem nicht an dem Gurt (2, 3, 4, 5, 13, 15), sondern in anderer Weise am Patienten angeordneten Sensor (12) elektrisch verbindbar ist.

2. Gurtsystem nach Anspruch 1, **dadurch gekennzeichnet, dass** alle oder mehrere der Steuerungs- und/oder Messleitungen (9, 9a, 9b, 9c, 9d, 9e) zu einem Anschluss (7) zusammengeführt sind, der mit einer externen Steuerungs- und/oder Auswerteinrichtung zur Signalübertragung verbindbar ist.

3. Gurtsystem nach Anspruch 2, **dadurch gekennzeichnet, dass** die Verbindung des Anschlusses (7) zur Signalübertragung mit der externen Steuerungs- und/oder Auswerteinrichtung mittels eines drahtlosen Übertragungsmodul erfolgt.

4. Gurtsystem nach einem der vorhergehenden Ansprüche, **dadurch gekennzeichnet, dass** eine Kabelführung für alle oder mehrere der Steuerungs- und/oder Messleitungen (9, 9a, 9b, 9c, 9d, 9e) innerhalb des Gurtes vorgesehen ist.

5. Gurtsystem nach einem der vorhergehenden Ansprüche, **dadurch gekennzeichnet, dass** das Gurtsystem einen Gurtsteg (15) umfasst, der mit mindestens einem anderen Gurt (2, 3) verbunden ist.

6. Gurtsystem nach Anspruch 5, soweit rückbezogen auf Anspruch 2, **dadurch gekennzeichnet, dass** in oder an dem Gurtsteg (15) der Anschluss (7) angeordnet ist.

7. Gurtsystem nach Anspruch 6, **dadurch gekennzeichnet, dass** der Anschluss (7) einen Anschlussstecker umfasst.

8. Gurtsystem nach einem der vorhergehenden Ansprüche, **dadurch gekennzeichnet, dass** das Gurtsystem einen Brustgurt (2), einen Bauchgurt (3) und einen Gurtsteg (15) umfasst, wobei der Gurtsteg (15) zwei Abschnitte umfasst, die einen unteren (5) und oberen Gurtsteg (4a, 4b) bilden, und der obere Gurtsteg (4a, 4b) mindestens mit dem Bauchgurt (3) und dem Brustgurt (2) und der untere Gurtsteg (5) mindestens mit dem Bauchgurt (3) verbunden ist.

9. Gurtsystem nach einem der Ansprüche 5 bis 8, **dadurch gekennzeichnet, dass** im am Patienten angelegten Zustand wenigstens ein Teil des Gurtstegs (4a, 4b, 5) im Wesentlichen in Richtung der longitudinalen Achse des Patienten ausgerichtet ist.

10. Gurtsystem nach einem der vorhergehenden Ansprüche, **dadurch gekennzeichnet, dass** eine oder mehrere der Steuerungs- und/oder Messleitungen (9, 9a, 9b, 9c, 9e) jeweils eine Anschlusssteckerbuchse (8) oder einen Anschlussstecker aufweisen, die mit einem dazu vorgesehenen und eingerichteten asymmetrischen Anschluss am Sensor (12) verbindbar sind.

11. Gurtsystem nach einem der vorangehenden Ansprüche, **dadurch gekennzeichnet, dass** der Sensor (12) an der Anordnungsstelle (10) mittels eines lösbaren Verschlussmittels, insbesondere eines Klettverschlusses, anordbar ist.

12. Gurtsystem nach einem der vorhergehenden Ansprüche, **dadurch gekennzeichnet, dass** mehr als eine Austrittstelle (11) vorgesehen ist und die Austrittstellen (11) in vorbestimmten Abständen, die von den Körpermaßen und dem Alter des Patienten (100) abhängig vorbestimmt sind, zueinander angeordnet sind.

13. Gurtsystem nach einem der vorhergehenden Ansprüche, **dadurch gekennzeichnet, dass** das Gurtsystem mehrere Widerstandstreifen umfasst, die in oder an mindestens einem Gurt (2, 3, 4a, 4b, 5, 13a, 13b, 15) angeordnet sind.

14. Gurtsystem nach Anspruch 13, **dadurch gekennzeichnet, dass** das Gurtsystem mehrere Gurte (2, 3, 4a, 4b, 5, 13a, 13b, 15) aufweist und in oder an mindestens zwei der Gurte (2, 3, 4a, 4b, 5, 13a, 13b, 15) jeweils wenigstens ein Widerstandstreifen angeordnet ist.

15. Gurtsystem nach Anspruch 13 oder 14, **dadurch gekennzeichnet, dass** die Widerstandstreifen Teil eines Impedanzmesskreises, insbesondere eines Impedanzmesskreises zur Messung der Atemtätigkeit des Patienten, sind.
